Europäisches Patentamt

European Patent Office

Office européen des brevets

(11) Publication number: **0 247 251 B1**

# EUROPEAN PATENT SPECIFICATION

(45) Date of publication of patent specification: 02.10.91

(51) Int. Cl.5: **A61K 9/02**, A61K 31/155, A61K 31/085

(21) Application number: 86202369.4

(22) Date of filing: 23.12.86

The file contains technical information submitted after the application was filed and not included in this specification

(54) Vaginal contraceptive.

(30) Priority: 01.02.86 NL 8600250

(43) Date of publication of application:
02.12.87 Bulletin 87/49

(45) Publication of the grant of the patent:
02.10.91 Bulletin 91/40

(84) Designated Contracting States:
AT BE CH DE ES FR GB GR IT LI LU NL SE

(56) References cited:
EP-A- 0 024 779   EP-A- 0 135 283
GB-A- 1 601 777   NL-A- 6 818 655
NL-A- 7 614 327   US-A- 4 031 202

CHEMICAL ABSTRACTS, vol. 103, no. 25, 23rd December 1985, page 144, abstract no. 206801r, Columbus, Ohio, US; S.M. LOUIS et al.: "A comparison of the effects of nonoxynol-9 and chlorhexidine on sperm motility", & CONTRACEPTION 1985, 32(2), 199-5

CHEMICAL ABSTRACTS, vol. 104, no. 19, 12th May 1986, page 71, abstract no. 162116k, Columbus, Ohio, US; D. SHARMAN et al.:

"Comparison of the action of nonoxynol-9 and chlorhexidine on sperm", & FERTIL. STERIL. 1986, 45(2), 259-64

(73) Proprietor: THE VICTORIA UNIVERSITY OF MANCHESTER
Oxford Road
Manchester. M13 9PL(GB)

(72) Inventor: CHANTLER, Eric N.
45, Longhurstlane, Mella, Stockport
SK1 1AA Cheshire(GB)

(74) Representative: Tunnicliffe, Peter Barry
Westfields House 5 Vincent Drive Lache Lane
Chester, CH4 7RQ(GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to a vaginal contraceptive, particularly to a diaphragm, containing two or more drugs, and to contraceptive compositions containing two or more drugs.

British patent specification No. 1,601,777 discloses a vaginal contraceptive, which is provided with a quantity of spermicidal cream or jelly, or other spermicides, prior to introduction into the vagina. This patent specification especially concerns the manufacture of diaphragms, whereas nothing is mentioned about the nature of the spermicide used.

The present invention is directed to improved contraceptive compositions and to a a vaginal contraceptive which functions better as a contraceptive than said prior art device.

Our invention is based on the use of at least two different spermicides, one of which is a non-ionic detergent of the alkyl phenol ethoxylate type and the other is a biguanide with antiseptic properties, to manufacture improved contraceptive devices and improved contraceptive compositions. To this end, the vaginal contraceptive according to the invention is characterised in that the contraceptive is impregnated with a mixture of at least two different spermicides in water.

Thus according to our invention we provide a vaginal contraceptive, particularly a diaphragm, containing two or more drugs, characterised in that the contraceptive is impregnated with a mixture of at least two different spermicides in water, one of which is a non-ionic detergent of the alkyl phenol ethoxylate type and the other is a biguanide with antiseptic properties.

According to our invention we also provide a contraceptive composition characterised in that it contains a mixture of at least two different spermicides, one of which is a non-ionic detergent of the alkyl phenol ethoxylate type and the other is a biguanide with antiseptic properties.

The spermicides used exhibit a synergistic effect, meaning that the combination of the spermicides used according to this invention possesses an activity exceeding the sum of the activities of the separate spermicides. One of the spermicides specified according to the invention exhibits a spermicidal activity mainly in the vagina while the other possesses a good spermicidal activity in the vagina as well as in the cervical mucus.

It has been found in practice that nonoxynol-9 is very satisfactory as the non-ionic detergent type of spermicide and chlorhexidine gluconate is very satisfactory as the biguanide type of spermicide.

When a combination of nonoxynol-9 and chlorhexidine gluconate is used, a very surprising synergistic contraceptive effect is obtained. This synergism becomes evident from the following experiment.

The use of 670 $\mu$g of chlorhexidine gluconate on 1 ml of ejaculate results in an immediate decrease in the motility of the spermatozoa by 50-60%, whereas 20% of the spermatozoa still retain their motility 15 minutes after addition.

It has been found that a concentration of 67 $\mu$g of nonoxynol-9 in 1 ml of ejaculate immediately inhibits the motility of the spermatozoa by 60%. This level of inhibition remains stable without further loss of spermatozoa motility.

Surprisingly it has been found that when a mixture of 670 $\mu$g of chlorhexidine gluconate and 67 $\mu$g of nonoxynol-9 is added to 1 ml of ejaculate, an immediate decrease of 75% of the motility of the spermatozoa occurs, resulting in a total loss of motility after only two minutes following addition of the said spermicides.

For the purposes of this invention, a mixture of spermicides as specified containing 10-15% vol./vol. (preferably 12% vol./vol.) of nonoxynol-9 and 0.5-1.5% by weight/vol. (preferably 1% by weight/vol.) of chlorhexidine gluconate, in water, yields good results.

In addition to spermicides one can also incorporate other conventional drugs into the mixture. Suitable drugs include, without limitation, fungicides, antibiotics, anti-inflammatories, etc., provided that these drugs are compatible with each other.

According to a further feature of the present invention we also provide a vaginal contraceptive which is packed in a liquid-tight (i.e. sealed) package together with a liquid composition containing the mixture of spermicides of the invention.

The vaginal contraceptive, particularly a diaphragm, when thus packed is thoroughly impregnated with the active ingredients prior to insertion into the vagina. Examples of such a liquid-tight packing include bags of synthetic material, aluminium foil, etc..

An important advantage of the impregnated contraceptive of the invention resides in the fact that, after its introduction into the vagina, the active ingredients can perform their action during a few days because of their sustained release, thereby achieving an almost 100% contraceptive effect.

Besides diaphragms it is also possible to use other current vaginal contraceptives, such as e.g. sponge-like devices, rings of synthetic material, etc..

The diaphragm normally used according to the invention is manufactured starting from a synthetic foam of a given thickness, which is compressed at elevated temperature and pressure. The product thus obtained, being porous, has the ability to absorb the mixture of spermicides in water and subsequently release said mixture slowly.

It is noted that the use of rings containing a medication is disclosed in Dutch patent application No. 68.18655. In this patent application the following substances are mentioned as drugs: digitoxin, triiodothyronine, isoproterenol, atropine, histamine, nitrogen mustard, vitamin $B_{12}$, pyrimethanine, hormonal substances, that is oestrogenic substances, progestational substances, androgenic substances, for example oestradiol, progesterone, androstenedione, testosterone, cortisol, medroxyprogesterone acetate, melengestrol acetate, chlormadinone, etc.. In this patent application nothing is mentioned about the combination of specific spermicides as in the present invention, or about a possible synergism.

Dutch patent application No. 76.14327 discloses a releasing device, which contains a biologically active substance and/or lubricant, the biologically active substance being for example a spermicide. However, in this application too no mention is to be found of the combination of the specific spermicides as in the present invention.

The invention will be described in more detail in the following example.

EXAMPLE

A diaphragm is packed in a liquid-tight manner in an envelope of synthetic material. In this envelope is contained a mixture comprising 12% vol./vol. of nonoxynol-9 and 1% weight/vol. of chlorhexidine gluconate, the balance being water. The packed diaphragm thus becomes impregnated with the spermicides contained in the mixture.

For use, the diaphragm thus impregnated is removed from the envelope, slightly shaken, and inserted into the vagina by the user, where the impregnated diaphragm can perform its contraceptive action during several days.

The scope of the present invention is not limited to this example. Other spermicides and contraceptives can be used without departing from the scope of the present invention.

**Claims**

1.  A vaginal contraceptive, particularly a diaphragm, containing two or more drugs, characterised in that the contraceptive is impregnated with a mixture of at least two different spermicides in water, one of which is a non-ionic detergent of the alkyl phenol ethoxylate type and the other is a biguanide with antiseptic properties.

2.  A vaginal contraceptive as claimed in Claim 1 or Claim 2 characterised in that the mixture of spermicides contains 10-15% vol./vol. of non-ionic detergent and 0.5-1.5% by weight/vol. of the biguanide, the balance being water.

3.  A vaginal contraceptive as claimed in Claim 1 or Claim 2, characterised in that the non-ionic detergent is nonoxynol-9.

4.  A vaginal contraceptive as claimed in any of Claims 1 to 3, characterised in that the biguanide is chlorhexidine gluconate.

5.  A vaginal contraceptive device which is packed in a liquid-tight (i.e. sealed) package together with a liquid composition of the two components as defined in Claim 1.

6.  A contraceptive composition characterised in that it contains a mixture of at least two different spermicides, one of which is a non-ionic detergent of the alkyl phenol ethoxylate type and the other is a biguanide with antiseptic properties.

7.  A contraceptive composition as claimed in Claim 6, characterised in that the non-ionic detergent is nonoxynol-9.

8.  A contraceptive composition as claimed in Claim 6 or Claim 7 characterised in that the biguanide is chlorhexidine gluconate.

9.  A contraceptive composition as claimed in any of Claims 6 to 8, characterised in that the components are in water.

10. Use of at least two different spermicides, one of which is a non-ionic detergent of the alkyl phenol ethoxylate type and the other is a biguanide with antiseptic properties, to manufacture a contraceptive composition.

11. Use of a non-ionic detergent of the alkyl phenol ethoxylate type to manufacture a contraceptive composition which comprises at least two different spermicides, one of which is a non-ionic detergent of the alkyl phenol ethoxylate type and the other is a biguanide with antiseptic properties.

12. Use of a biguanide with antiseptic properties to manufacture a contraceptive composition which comprises at least two different spermicides, one of which is a non-ionic detergent of the alkyl phenol ethoxylate type and the other is a biguanide with antiseptic properties.

13. Use of nonoxynol-9 according to any of Claims 10 to 11.

14. Use of chlorhexidine gluconate according to any of Claims 10 to 13.

**Revendications**

1. Un contraceptif vaginal, en particulier un diaphragme, contenant deux ou plusieurs principes actifs, caractérisé en ce que le contraceptif est imprégné d'un mélange aqueux d'au moins deux spermicides différents, l'un étant un détergent non-ionogène du type éthoxylate d'alkyl-phénol et l'autre un biguanide ayant des propriétés antiseptiques.

2. Un contraceptif vaginal selon la revendication 1 ou 2, caractérisé en ce que le mélange de spermicides contient 10 à 15 % en volume de détergent non-ionogène et 0,5 à 1,5 %, en poids par volume, de biguanide, le reste étant de l'eau.

3. Un contraceptif vaginal selon la revendication 1 ou 2, caractérisé en ce que le détergent non-ionogène est le nonoxynol-9.

4. Un contraceptif vaginal selon l'une des revendications 1 à 3, caractérisé en ce que le biguanide est le gluconate de chlorhexidine.

5. Un dispositif contraceptif vaginal qui est emballé dans une enveloppe imperméable aux liquides (par exemple scellée), conjointement avec une composition liquide des deux constituants définis dans la revendication 1.

6. Une composition contraceptive, caractérisée en ce qu'elle contient un mélange d'au moins deux spermicides différents, l'un étant un détergent non-ionogène du type éthoxylate d'alkyl-phénol et l'autre un biguanide ayant des propriétés antiseptiques.

7. Une composition contraceptive selon la revendication 6, caractérisée en ce que le détergent non-ionogène est le nonoxynol-9.

8. Une composition contraceptive selon la revendication 6 ou 7, caractérisée en ce que le biguanide est le gluconate de chlorhexidine.

9. Une composition contraceptive selon l'une des revendications 6 à 8, caractérisée en ce que lesdits constituants sont sous forme d'un mélange aqueux.

10. L'utilisation d'au moins deux spermicides, l'un étant un détergent non-ionogène du type éthoxylate d'alkyl-phénol et l'autre un biguanide ayant des propriétés antiseptiques, pour la fabrication d'une composition contraceptive.

11. L'utilisation d'un détergent non-ionogène du type éthoxylate d'alkyl-phénol pour fabriquer une composition contraceptive qui comprend au moins deux spermicides différents, l'un étant un détergent non-ionogène du type éthoxylate d'alkyl-phénol et l'autre un biguanide ayant des propriétés antiseptiques.

12. L'utilisation d'un biguanide ayant des propriétés antiseptiques pour fabriquer une composition contraceptive qui comprend au moins deux spermicides différents, l'un étant un détergent non-ionogène du type éthoxylate d'aikyl-phénol et l'autre un biguanide ayant des propriétés antiseptiques.

13. L'utilisation du nonoxynol-9 selon l'une des revendications 10 ou 11.

14. L'utilisation du gluconate de chlorhexidine selon l'une des revendications 10 à 13.

**Patentansprüche**

1. Ein vaginales Empfängnisverhütungsmittel, insbesondere ein Diaphragma, das zwei oder mehr Wirkstoffe enthält, dadurch gekennzeichnet, daß das Empfängnisverhütungsmittel mit einer Mischung aus mindestens zwei verschiedenen Spermiziden in Wasser imprägniert ist, wovon das eine ein nichtionogenes Tensid des Typs Alkylphenolethoxylat und das andere ein Biguanid mit antiseptischen Eigenschaften ist.

2. Ein vaginales Empfängnisverhütungsmittel, wie in Anspruch 1 oder Anspruch 2 beansprucht, dadurch gekennzeichnet, daß die Mischung der Spermiziden 10-15 Vol. % eines nichtionogenen Tensids und 0,5-1,5 % Gew./Vol. des Biguanid enthält und der Rest Wasser ist.

3. Ein vaginales Empfängnisverhütungsmittel, wie in Anspruch 1 oder Anspruch 2 beansprucht, dadurch gekennzeichnet, daß das nichtionogene Tensid Nonoxynol-9 ist.

4. Ein vaginales Empfängnisverhütungsmittel, wie in irgendeinem der Ansprüche 1 bis 3 beansprucht, dadurch gekennzeichnet, daß das Biguanid Chlorhexidinglukonat ist.

5. Ein vaginales Empfängnisverhütungsmittel, das in einer flüssigkeitsundurchlässigen (d. h. versiegelten) Packung verpackt ist zusammen mit einer flüssigen Zusammensetzung der beiden Bestandteile, wie in Anspruch 1 definiert.

6. Eine empfängnisverhütende Zusammensetzung, dadurch gekennzeichnet, daß sie eine Mischung von mindestens zwei verschiedenen Spermiziden enthält, wovon eines ein nichtionogenes Tensid des Typs Alkylphenolethoxylat und das andere ein Biguanid mit antiseptischen Eigenschaften ist.

7. Eine empfängnisverhütende Zusammensetzung, wie in Anspruch 6 beansprucht, dadurch gekennzeichnet, daß das nichtiogene Tensid Nonoxynol-9 ist.

8. Eine empfängnisverhütende Zusammensetzung, wie in Anspruch 6 oder Anspruch 7 beansprucht, dadurch gekennzeichnet, daß das Biguanid Chlorhexidinglukonat ist.

9. Eine empfängnisverhütende Zusammensetzung, wie in irgendeinem der Ansprüche 6 bis 8 beansprucht, dadurch gekennzeichnet, daß die Bestandteile sich in Wasser befinden.

10. Verwendung von mindestens zwei verschiedenen Spermiziden, wovon das eine ein nichtiogenes Tensid des Typs Alkylphenolethoxylat und das andere ein Biguanid mit antiseptischen Eigenschaften ist, um eine empfängnisverhütende Zusammensetzung herzustellen.

11. Verwendung eines nichtiogenen Tensids des Typs Alkylphenolethoxylat, um ein empfängnisverhütendes Gemisch herzustellen, das mindestens zwei verschiedene Spermizide enthält, wovon das eine ein nichtiogenes Tensid des Typs Alkylphenolethoxylat und das andere ein Biguanid mit antiseptischen Eigenschaften ist.

12. Verwendung eines Biguanid mit antiseptischen Eigenschaften, um eine empfängnisverhütende Zusammensetzung herzustellen, die mindestens zwei verschiedene Spermizide enthält, wovon das eine ein nichtiogenes Tensid des Typs Alkylphenolethoxylat und das andere ein Biguanid mit antiseptischen Eigenschaften ist.

13. Verwendung von Nonoxynol-9 gemäß irgendeinem der Ansprüche 9 bis 11.

14. Verwendung von Chlorhexidinglukonat gemäß irgendeinem der Ansprüche 10 bis 13.